# EUROPEAN PATENT SPECIFICATION

(11) **EP 1 714 645 B1**
(45) Date of publication and mention of the grant of the patent: **15.10.2008**
(21) Application number: 05008886.3
(22) Date of filing: 22.04.2005
(51) Int. Cl.: A61K 31/05, A61P 19/10, A61K 36/18, A61P 35/00

(54) **Pharmaceutical composition containing bakuchiol for treating breast cancer in women**
Bakuchiol enthaltende pharmazeutische Zusammensetzung zur Behandlung von weiblichem Brustkrebs
Composition pharmaceutique contenant du bakuchiol pour traiter le cancer du sein chez la femme

(43) Date of publication of application: 25.10.2006
(73) Proprietor: Sinphar Pharmaceutical Co., Ltd., Taipei (TW)
(72) Inventor: Lin, Hang-Ching, Taipei (TW); Ding, Hsiou-Yu, Tainan (TW); Chang, Wen-Liang, Taipei (TW); Chao, Chien-Lian, Taoyuan (TW); Huang, Hsin-Wen, Sanmim Distict Kaohsiung (TW)
(74) Representative: Grünecker, Kinkeldey, Stockmair & Schwanhäusser Anwaltssozietät

(56) References cited:
- KRENISKY JOANN M ET AL: "Isolation and antihyperglycemic activity of bakuchiol from Otholobium pubescens (Fabaceae), a peruvian medicinal plant used for the treatment of diabetes" BIOLOGICAL AND PHARMACEUTICAL BULLETIN, vol. 22, no. 10, October 1999 (1999-10), pages 1137-1140, XP008052467 ISSN: 0918-6158
- DATABASE WPI Section Ch, Week 199110 Derwent Publications Ltd., London, GB; Class B05, AN 1991-070474 XP002345848 & JP 03 020218 A (KYORITSU YAKUHIN KO) 29 January 1991 (1991-01-29)
- SHI YONG RYU ET AL: "ANTITUMOR ACTIVITY OF PSORALEA CORYLIFOLIA" ARCHIVES OF PHARMACAL RESEARCH, NATL. FISHERIES UNIVERSITY, PUSAN, KR, vol. 15, no. 4, 1992, pages 356-359, XP008052399 ISSN: 0253-6269
- RANGARI V D ET AL: "CHEMISTRY & PHARMACOLOGY OF PSORALEA CORYLIFOLIA" INDIAN DRUGS, XX, XX, vol. 29, no. 15, 1992, pages 662-670, XP008052406
- KUBO M ET AL: "CYTOTOXICITY OF CORYLIFOLIAE FRUCTUS. I. ISOLATION OF THE EFFECTIVE COMPOUND AND THE CYTOTOXICITY" YAKUGAKU ZASSHI - JOURNAL OF THE PHARMACEUTICAL SOCIETY OF JAPAN, NIHON YAKUGAKKAI, TOKYO,, JP, vol. 109, no. 12, 1989, pages 926-931, XP008052398 ISSN: 0031-6903
- DATABASE CA [Online] CHEMICAL ABSTRACTS SERVICE, COLUMBUS, OHIO, US; 18 July 1995 (1995-07-18), IINUMA, MUNEKAZU ET AL: "Psoralea corylifolia extracts as bone strength-enhancing and calcifying agents for therapeutic use" XP002345840 retrieved from STN Database accession no. 1995:680889 & JP 07 109225 A2 (MARUHO KK, JAPAN) 25 April 1995 (1995-04-25)

## Description

### Field of the Invention:

The present invention relates to a pharmaceutical composition containing bakuchiol as an effective ingredient for the prevention or treatment of a woman suffering breast cancer or post-menopausal osteoporosis. The present invention also relates to an extract containing bakuchiol capable of preventing or treating a woman suffering breast cancer or osteoporosis.

### Background of the Invention:

Psoraleae fructus is a ripe fruit of *Psoralea corilifolia* and is traditionally used as a tonic in Chinese herbal medicine. Psoraleae fructus contains many chemical ingredients which have been found possessing pharmacological activities as shown in the literature. Beside lipids, the main chemical ingredients contained in psoraleae fructus include psoralen, isopsoralen and bakuchiol. Among the ingredients, bakuchiol with a phenol terpene structure has received a lot of attention in the past. Many articles related to bakuchiol reveal that bakuchiol has the pharmacological activities as follows: antimutation, hepatoprotection, antioxidation, weak estrogen like effect, cytotoxic effect, DNA polymerase inhibitor, anti-inflammation, and antihyperglycemic effect.

Among the patent disclosures, Japan patent publication No. 11-71231 discloses that bakuchiol is capable of inhibiting tyrosinase and can be used in making cosmetics having a skin whitening effect. Japan patent publication Nos. 2000-327581 and 2001-233707 disclose the bacteriostatic effects of bakuchiol, and its use as an agent in sterilizing oral cavity, anti-legionella agent, and anti-MRSA agent. Japan patent publication No. 3-20218 discloses the cellular toxicity of bakuchiol and its use as an anti-corn agent. Japan Patent 7-109225 discloses that lipids in psoraleae fructus are effective in strengthening bone strength through bone calcification. Current data show that the use of estrogen in treating postmenopausal estrogen-deficient osteoporosis will increase the probability of the subject woman in gaining breast cancer. Thus, there is a pressing need in developing a medicine capable of treating postmenopausal estrogen-deficient osteoporosis without increasing the probability of the subject woman in gaining breast cancer. Of cause, a medicine capable of treating breast cancer, as well as postmenopausal estrogen-deficient osteoporosis is also beneficial.

### Summary of the Invention:

The main objective of the present invention is to provide a bakuchiol compound or an extract containing bakuchiol in the prevention or treatment of breast cancer. An embodiment of such a use is a pharmaceutical composition for the prevention or treatment of breast cancer. A pharmaceutical composition according to the present invention contains bakuchiol or an extract containing bakuchiol in the prevention or treatment of breast cancer, which can be in the dosage forms of topical use, oral administration, injection or sustained release.

Another objective of the present invention is to provide a novel use of bakuchiol or an extract containing bakuchiol in the prevention or treatment of breast cancer and osteoporosis. An embodiment of this novel use is a pharmaceutical composition containing bakuchiol or the extract, which can be in the dosage forms of topical use, oral administration, injection or sustained release.

An in vitro cell cultivation test according to the present invention indicates that bakuchiol even at a low concentration has the effect of inhibiting the growth of breast cancer cells. This result shows that bakuchiol has a selective inhibition effect on some human breast cancer cells and is suitable to be used as a therapeutic or preventative agent for breast cancer. This finding on the effect of bakuchiol on human breast cancer cells is different from a previous finding by a Korean scholar: Toxicity of bakuchiol on five different human cancer cells (lung (A549), ovary (SK-OV-3), skin (SK-MEL-2), central nervous system (XF498), and large intestine (HCT)), with an average inhibitory concentration (IC₅₀) of 10∼15 µg/cc (Arch. Pharm. Res. 15, 356-359 (1992)). The average inhibitory concentrations of these five cancer cells being in such a narrow range indicates that cytotoxicity of bakuchiol is not selective to those five cancer cells. Furthermore, that research does not include human breast cancer cells.

Furthermore, the present invention includes animal tests. The ovary of a female rat was removed to simulate osteoporosis caused by estrogen deficiency. The result shows that bakuchiol at a low dose (5 mg/kg) possess a therapeutic effect on osteoporosis by inhibiting the bone resorption. This finding is different from a previous finding by a Japanese scholar: Lipids in Psoraleae fructus only enhance bone calcification and show no enhancement on bone mass (Japan patent publication No. 7-109225 and Planta med. 62, 150-153 (1996)). The best treatment on osteoporosis lies in a situation where a medicine can promote bone formation and/or inhibit bone resorption. The function of bakuchiol is similar to that of estrogen which includes prevention and treatment of bone resorption. This function obviously is superior to the bone calcification function of the lipids.

From another point of view, the use of estrogen in the treatment of postmenopausal osteoporosis increases the risk of breast cancer. Bakuchiol has the effects in preventing or treating osteoporosis, as well as toxic effect to breast cancer cells. Thus, bakuchiol has the potential in being used as a medicine in preventing or treating a woman suffering from breast cancer/osteoporosis.

Prior art shows that an extract of *Psoraleae corilifolia* can be separated into lipids and bakuchiol by silica gel column chromatography. Since the chemical properties thereof are different, they can be easily separated by using thin layer chromatography (TLC), subsequently, lipids cannot be seen under an UV lamp but can be detected by an iodine vapor, and bakuchiol can be easily detected by an UV lamp. Therefore, as shown in the following Example 1, only simple eluting agent is used to purify and separate lipids and/or bakuchiol.

### Detailed Description of the Invention:

Preferred embodiments of the present invention includes the following:
1. Use of bakuchiol having the following formula (I) or a pharmaceutically acceptable ester or salt thereof in the manufacture of a medicament for treating human breast cancer in a woman patient: 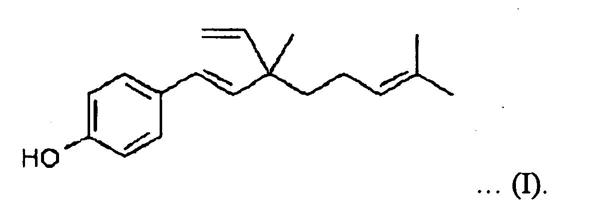
2. The use as described in Item 1, wherein said medicament is to be administered through topic application, injection, oral administration, or time release dosage form.
3. The use as described in Item 2, wherein said medicament is to be administered orally.
4. Use of bakuchiol having the formula (I) or a pharmaceutically acceptable ester or salt thereof in the manufacture of a medicament for treating human breast cancer and woman osteoporosis in a woman patient.

A process for preparing an extract containing bakuchiol according to one of the preferred embodiments of the present invention comprises the following steps:
a) Using an organic solvent, selected from the group consisting of n-hexane, acetone, ethyl acetate, methanol, ethanol, and a mixture thereof, to extract Psoraleae fructus in the form of a powder by grinding;
b) Concentrating the resulting solution from the extraction, separating the concentrated solution with silica gel column chromatography by using a mixed solution of n-hexane and ethyl acetate (n-hexane : ethyl acetate = 9 : 1) as an eluting agent to sequentially obtain an eluate containing (1) lipids and an eluate containing (2) bakuchiol, or collect an eluate containing both the (1) lipids and (2) bakuchiol. After concentrated, the eluate becomes an extract containing bakuchiol, or an extract containing (1) lipids and (2) bakuchiol.

The eluate obtained in step (b) is identified by a thin layer chromatography (TLC), wherein a mixed solution of n-hexane and ethyl acetate (n-hexane : ethyl acetate = 9 : 1) is used as a mobile phase to develop the eluate , and an UV lamp or an iodine vapor is used to identified the lipids and bakuchiol. The chromatography value (R_{f}) for the eluate containing lipids is greater than 0.29 (R_{f}> 0.29), while the value for the eluate containing bakuchiol is 0.29 (R_{f} = 0.29).

The present invention can be better understood through the following examples, which are illustrative of the present invention.

### Example 1:

Three hundred grams of Psoraleae fructus powder was extracted by 2.4 L of acetone, and the mixture was separated into solid and liquid phases. The extraction and solid/liquid separation were repeated three times. The filtrates were combined, and the solvent was removed by evaporation to obtain 72.11 g of an oily extract. Twenty two of said oily extract was separated by a silica gel column (9.6 x 25 cm) packed with 300 g of silica gel (Merck Co., Silica gel 60, mesh 70~230). A mixture of n-hexane/ethyl acetate (9 : 1), acetone, and methanol were sequentially used as an eluent to elute the column. Twenty five bottles of eluate of n-hexane/ethyl acetate (9:1) were first collected from the column. Furthermore, 10 bottles (Bottle Nos. 26-35) of eluate of acetone were collected from column, followed by 10 bottles (Bottle Nos. 36-45) of eluate of methanol were collected from the column. Every 300 ml of the eluate was collected separately, i.e. 300 ml per bottle. The eluate was identified by a silica gel thin layer chromatography (TLC) developed by a mixed solution of n-hexane/ethyl acetate (9:1) using an UV lamp or an iodine vapor. The bottles of the eluate containing same ingredients analyzed by TLC were combined.

Eluate in the bottle Nos. 1-7 shows no UV absorption spot on the TLC plate, indicating that the eluate contains lipids. The eluate in bottles of No. 1-7 was combined and concentrated to obtain 6.116 g of oily substances. Eluate in the bottle of No. 8-20 show only one UV absorption spot on the TLC plate (R_{f}= 0.29). The eluate in the bottles of No. 8-20 was combined and concentrated to obtain 4.543 g of the oily bakuchiol. The eluate in bottles of No. 21-45 containing no bakuchiol was combined and concentrated to obtain 11.04 g of substances. The above-mentioned data indicate that: a total of 240.36 g of extract can be obtained per kg of *Psoralea corilifoli*, i.e. an extraction ratio of 24.04%. The 240.36 g of extract contains 66.82 g of lipids (6.68%, based on the weight of Psoraleae fructus), 49.64 g of bakuchiol (4.96%, based on the weight of Psoraleae fructusi), and 123.9 g of other Psoraleae fructus ingredients (extraction ratio of 12.4%, based on the weight of *Psoraleae corilifoli*).

The structure of bakuchiol was identified with the following data: [α]²⁷, D⁺ 24° (C 1.0 , CHCl3); EI-MS m/z (rel. int. %): 256 ([m]⁺, 24), 173 (100); UV (EtOH) λmax (log ε): 260 nm (4.26); IR (KBr) υmax 3350, 1650, 1530, 1245, 1010, 980, 922 cm⁻¹; ¹³C-NMR (δ, CDCl3): C-1 (25.6), C-2 (131.2), C-3 (124.8), C-4 (23.2), C-5 (41.2), C-6 (42.5), C-7 (135.7), C-8 (126.5), C-9 (130.7), C-10 (127.3), C-11 (115.4), C-12 (154.6), C-13 (115.4), C-14 (127.3), C-15 (23.3), C-16 (145.9), C-17 (111.8), C-18 (17.6). ¹H-NMR (δmult. (J in Hz), CDCl3): H-1 (1.61,S), H-3 (5.15, t (7.3)), H-4 (1.99, q (7.3)), H-5 (1.53, m), H-7 (6.08,d (16.2)), H-8 (6.28, d (16.2)), H-10 (7.26, d (8.5)), H-11 (6.80, d (8.5)), H-13 (6.80, d (8.5)), H-14 (7.26, d (8.5)), H-15 (1.23, S), H-16 (5.91, dd (10.7, 17.4)), H-17 (5.06, m), H-18 (1.71,S)

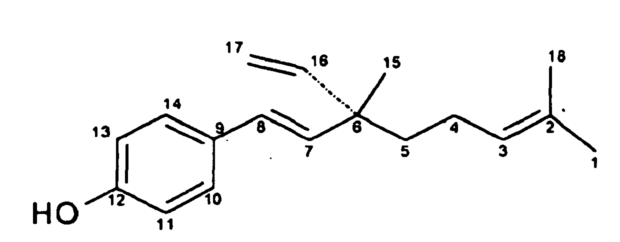

### Example 2:

### Cytotoxicity of bakuchiol in vitro

Materials: DMEM (medium), RPMI (medium), L-glutamine, penicillin, streptomycin and FBS (fetal bovine serum) were obtained from Gibco (NY, USA). Culture plasticware was purchased from Coming (MA, USA). Tamoxifen was obtained from Sigma-Aldrich (MO, USA), CCK-8 (Cell counting Kit, a new water soluble tetrazolium salt) was obtained from Dojindo Molecular Technologies (MD, USA). All other chemicals used were of reagent grade and were purchased from Sigma or E. Merck (Germany).

Cell lines and cell cultures: The human breast cancer cell lines T47D and MDA-MB231 were obtained from American Type Culture Collection (ATCC). Of both cancer cells, T47D is estrogen receptor positive, and MDA-MB231 is estrogen receptor negative. Cells were cultured at 37°C in RPMI medium containing 10% charcoal-dextran stripped fetal bovine serum for T47D cells and in DMEM medium containing 10% charcoal-dextran stripped fetal bovine serum for MDA-MB231 cells with 5% CO₂. The cell proliferation was determined by counting the viable cells with trypan blue exclusion.

Cytotoxicity assay: Dispense 198 µl of cell suspension (20,000 cells for T47D per well and 10,000 cell for MDA-MB231 per well) in a 96-well plate and pre-incubate the plate for 24 hours in an incubator at 37°C with humidified 5% CO₂. Then, add 2 µl of various concentrations of tamoxifen, bakuchiol and DMSO (control group) into the culture media in the plate and every concentration was repeatedly added six times. After plate was incubated for 48 hours in the incubator, media were aspirated of and new media (100 µl) were added and incubated for 1 more hour. Add 5 µl of CCK-8 solution to each well of the plate and incubate the plate for 4 hours in the incubator. Measure the absorbance at 450 nm using a ELISA reader. Cytotoxicity of test materials at various concentrations was estimated as the net growth % of cells compared with that of the control group (without test material, net growth = 100%). The dose-response curve of test materials were constructed and the IC₅₀ value was calculated as the concentration of test material that cause 50% inhibition of cell growth. The result of cytotoxicity of bakuchiol was shown in Table 1.

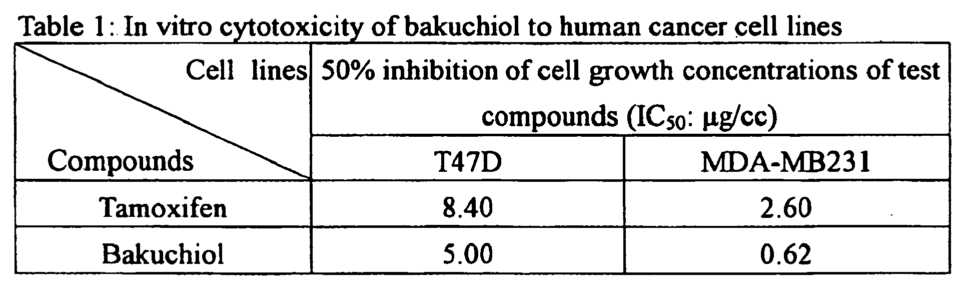

As it can be seen from Table 1 that IC₅₀ concentrations of bakuchiol are 5.00 and 0.62µg/cc to human breast cancer cell lines T47D and MDA-MB231, respectively. When human breast cancer cells (T47D/MDA-MB231), with above mentioned IC₅₀ concentrations of bakuchiol, were compared with five different human cancer cells, lung (A549), ovary (SK-OV-3), skin (SK-MEL-2), central nervous system (XF498), and large intestine (HCT), with an average inhibitory concentration (IC₅₀) of 10∼15 µg/cc of bakuchiol (Arch. Pharm. Res. 15, 356-359 (1992), it indicate that bakuchiol is selective and potent in inhibiting the growth of human breast cancer cell lines. Further, bakuchiol is also superior in inhibiting the growth of human breast cancer cell lines in comparison with Tamoxifen, a drug clinically used for human breast cancer, as shown in Table 1.

### Example 3:

The purpose of this example is to show the potency of bakuchiol in treating osteoporosis, wherein ovary of a female rat is removed to stimulate osteoporosis caused by estrogen deficiency.

### ANIMAL WORK

The female Wistar rats of 16 weeks old (unless specified in the other experiment description) were obtained from the stock reared by the Animal breeding center, National Scientific Council in Taipei. These rats were grouped by weights matched in order to distribute the physiological response variation from rats evenly into each group. These rats were grouping as follows:
Baseline group fed with no drug added food (n=8)
Sham operation group fed with no drug added food (Sham) (n=10)
Ovariectomy operation group fed with no drug added food (OVX) (n=8)
Ovariectomy operation group fed with Drug A (5 mg/kg) added food (OVX+A) (n=9)
Ovariectomy operation group fed with Drug B (15 mg/kg) added food (OVX+B) (n=9)
Ovariectomy (removal of ovaries) operation: Ovariectomy was induced using a protocol similar to that described by Wronski et al (Wronski et al, Endocrinology, 123(2), 681-86 (1988)).

During the experiments, the rats were housed in hanging grid cages in groups of two at 21°C with a 12 hours dark cycle. Food (Rat-mouse diet III, adequate in 1 % of calcium content and 1.2 % of phosphate content, Purina, USA) was administered by paired feeding between the experimental and control groups. Pair-feeding was carried out by calculating the average amount of food intake in the Sham group. So give the same amount of food to the OVX , OVX+A (5 mg/kg), OVX+B (15 mg/kg) group animals the following day. These animals were fed with a period of two months except for the baseline group which were killed on the very day of doing ovariectomy or sham operation. Rats of OVX+A and OVX+B groups were fed with the usual food chuck but added certain amount of compound A and B in it respectively. Water was given *ad libitum* to all rats.

### ADMINISTRATION OF TEST DRUGS

The test drug was weighted according to dose of 5 mg/kg and 15 mg/kg, and added into the ususal food chuck powder and mixed with 10% starch paste. After fully mixed, these chuck powder were made into mass manually and dried by air flow oven. The food with added drug to rats was performed by single blind method.

### IN VIVO BONE LABELLING PROCEDURE AND BONE HISTOMORPHOMETRY ASSESSEMENT

These labeling and bone histomorphometry assessment were done based on standard procedure in bone research (Lin et al, Calcif. Tissue Int., 67, 373-377 (2000)). Specimen were prestained with Villanueva bone stain, then decalcified and embedded in London resin. Undecalcified sections of 7 µm thick were cut using Jung-K microtome (Leica Ltd, USA) and treated with Villanueva stain for observation under light microscopy. Seven µm thick unstained sections were prepared for fluorescent microscopy and assessed with program Osteomeasure (3.0 version, Atlanta, USA). The bone volume (Table 2) and eroded surface parameters (Table 4) were obtained according to Lin et al. (Lin et al, Calcif. Tissue Int., 67, 373-377 (2000)). The experiment was performed under specific project licences from the Animal Center and IAACU, National Defense Medical Center, National Defense University, Taipei, Taiwan.

### APPARENT BONE DENSITY MEASUREMENT

The whole tibia and femur were stripped off attached muscles and ligaments after sacrifice. An apparent bone density measurement was done based on Archimedes floating principle on an electronic balance set with densitometry frame (Danielsen et al., Calcif. Tissue Int., 52, 26-33 (1993)). The weight of bone measured in the air was marked as W-air. The weight of the same bone weighed in water will be marked as W-water. The apparent bone density was calculated as [W-air/(W-air minus W-water)] (gm/cm³) (Table 3, Bone apparent density of Tibia).

**Table 2: Effect of bakuchiol on bone volume of proximal tibia head trabecular bone**

| Bone volume % (BV/TV)* | | | | |
|---|---|---|---|---|
| Baseline group | Sham group | OVX group | OVX+A group (5 mg/kg) | OVX+B group (15 mg/kg) |
| 27.50% | 29.50% | 12.50% | 19.80% | 20.11% |

| | | | | |
|---|---|---|---|---|
| *Mean value | | | | |

**Table 3: Effect of bakuchiol on bone apparent density of tibia**

| Bone apparent density (gm/cm³)* | | | | |
|---|---|---|---|---|
| Baseline group | Sham group | OVX group | OVX+A group (5 mg/kg) | OVX+B group (15 mg/kg) |
| 1.572 | 1.589 | 1.549 | 1.582 | 1.587 |

| | | | | |
|---|---|---|---|---|
| *Mean value | | | | |

**Table 4: Effect of bakuchiol on erosion surface (ES/BS, %) of proximal tibia**

| Erosion surface (ES/BS, %)* | | | | |
|---|---|---|---|---|
| Baseline group | Sham group | OVX group | OVX+A group (5 mg/kg) | OVX+B group (15 mg/kg) |
| 4.9% | 4.7% | 5.8% | 5.0% | 4.8% |

| | | | | |
|---|---|---|---|---|
| *Mean value | | | | |

### DATA ANALYSIS

These results were expressed as mean value and were transferred to the statistical package of SPSS 8.0 (SPSS Inc, Chicago, USA) for further data processing. The Bonferonni significant difference method was used for postHoc testing the multiple comparisons in one-way ANOVA after it had been showed to be statistical significance.

Table 2 and Table 3 show that the increase in the bone volume and the apparent bone density measured from the OVX+A and OVX+B groups (fed with bakuchiol added) in comparison with the OVX group (fed with no drug added) have statistical significance with p < 0.001 and p < 0.05, respectively. Accordingly, bakuchiol is effective in treating osteoporosis caused by post-menopausal estrogen deficiency. The potency mechanism of bakuchiol in treating osteoporosis can be verified by eroded surface parameters. As shown in Table 4, rats in the OVX+A and OVX+B groups (fed with bakuchiol added) show the inhibition of bone erosion (bone resorption) by the osteoclast with p < 0.001 in comparison with the OVX group (fed with no drug added).

## Claims

1. Use of bakuchiol having the following formula (I) or a pharmaceutically acceptable ester or salt thereof in the manufacture of a medicament for treating human breast cancer in a woman patient:

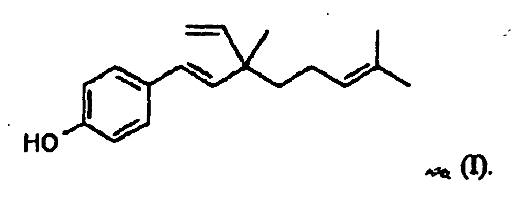

2. The use as claimed in Claim 1, wherein said medicament is to be administered through topic application, injection, oral administration, or time release dosage form.

3. The use as claimed in Claim 2, wherein said medicament is to be administered orally.

4. Use of bakuchiol having the following formula (I) or a pharmaceutically acceptable ester or salt thereof in the manufacture of a medicament for treating human breast cancer and woman osteoporosis in a woman patient:

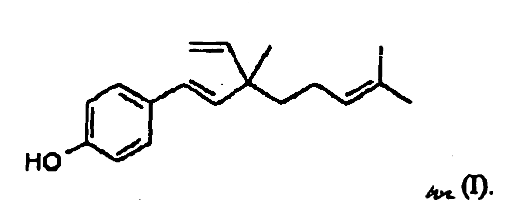

## Patentansprüche

1. Verwendung von Bakuchiol mit der folgenden Formel (I) oder einem pharmazeutisch verträglichen Ester oder Salz davon bei der Herstellung eines Medikaments zum Behandeln von menschlichem Brustkrebs bei einem weiblichen Patienten:

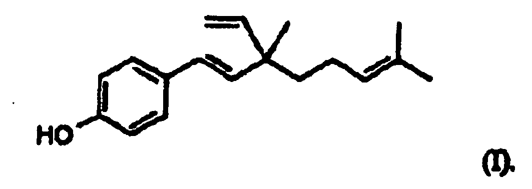

2. Verwendung nach Anspruch 1, wobei das Medikament durch topische Applikation, Injektion, orale Verabreichung oder eine Dosierungsform mit zeitlich festgelegter Freigabe verabreicht werden soll.

3. Verwendung nach Anspruch 2, wobei das Medikament oral verabreicht werden soll.

4. Verwendung von Bakuchiol mit der folgenden Formel (I) oder einem pharmazeutisch verträglichen Ester oder Salz davon bei der Herstellung eines Medikaments zum Behandeln von menschlichem Brustkrebs und Osteoporose von Frauen bei einem weiblichen Patienten:

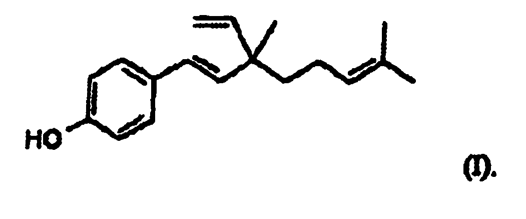

## Revendications

1. Utilisation de bakuchiol ayant la formule (I) suivante ou d'un ester ou sel pharmaceutiquement acceptable de celui-ci, dans la fabrication d'un médicament pour traiter un cancer du sein humain chez une patiente :

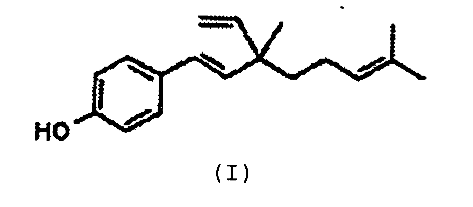

2. Utilisation selon la revendication 1, dans laquelle ledit médicament est destiné à être administré par application topique, par injection, par administration par voie orale, ou sous une forme posologique à libération prolongée.

3. Utilisation selon la revendication 2, dans laquelle le médicament est destiné à être administré par voie orale.

4. Utilisation de bakuchiol ayant la formule (I) suivante ou d'un ester ou sel pharmaceutiquement acceptable de celui-ci, dans la fabrication d'un médicament pour traiter le cancer du sein humain et l'ostéoporose féminine chez une patiente :
